# EUROPEAN PATENT APPLICATION

(11) **EP 2 198 862 A1**
(43) Date of publication of application: **23.06.2010**
(21) Application number: 08022020.5
(22) Date of filing: 18.12.2008
(51) Int. Cl.: A61K 31/353, C07D 311/32, A61P 33/06

(54) **Use of flavonoids to treat parasitic infection**

(71) Applicant: Citrox Limited, Kimbolton Cambridgeshire PE28 0NJ (GB)
(72) Inventor: Darlington, Gail, Surrey, KT20 6NR (GB); Ripley, Ian, Middlesbrough, TS7 8DP (GB)
(74) Representative: Sahans, Sarah Anne

(57) **Abstract**

The present invention relates to flavonoids and to flavonoid-containing compositions for use in the treatment of parasites, in particular for the treatment of malaria and lice.

## Description

The present invention relates to flavonoids and to flavonoid-containing compositions for use in the treatment of parasites, such as internal parasites more particularly to protozoal parasites such as malaria and especially by oral administration, and in the treatment of external parasites such as lice and in particular head lice.

In developing countries, in addition to viral and bacterial diseases such as HIV/AIDS and tuberculosis, millions of people are exposed to internal parasites which cause high levels of mortality, morbidity in humans and farmed and companion animals due to those parasites. Widespread concern exists since the efficacy of current drugs is seriously diminished by the development of widespread drug resistance. Even in developed countries parasites in humans and mammals such as farmed and companion animals are a significant problem, for example with humans returning from areas where certain parasitic infections, such as malaria, are endemic.

Flavonoids are a group of chemicals naturally occurring in plants which have significant antimicrobial and antioxidant activities. They occur in higher plants, including gymnosperms, ferns and mosses. There is enormous diversity throughout the plant kingdom. Many of these flavonoids are highly coloured and constitute the majority of red, yellow and blue pigments in flowers and fruits (other than carotenoids and a few coloured alkaloids).

Flavonoid compounds have huge diversity throughout the plant kingdom and may be divided into several major groups as follows:-

| | |
|---|---|
| Flavones | Flavanones |
| Flavanols | Flavanonols |
| Isoflavones | Anthocyanins |
| Anthocyanidins | Leucoanthocyanins |
| Chalcones | Dihydrochalcones |
| Aurones | Catechins |

The evidence for flavonoid-rich foods being cardioprotective, neuroprotective and chemoprotective is steadily accumulating, although their mechanisms and actions still remain largely to be elucidated. Although there are hundreds of flavonoid molecules in dietary plants, the major components of current interest for their beneficial health effects are members of the flavonol, flavanol, flavanone, anthocyanin and hydroxycinnamate families, as well as specific stilbene and chalcone structures. Molecules of interest are the flavanol components of grapes, wine, teas and berry fruits in the berry fruit flavonoids, including the green tea constituents repigallo catechins, resveratrol and quercetin.

Flavonoid research is a major area of current interest and in particular the field has gained momentum through the knowledge that flavonoids are modified during metabolism in the intestines and by the actions of the colonic microflora and in conjunction with hepatic metabolism. Thus the flavonoid forms reaching the cells and tissues after leaving the gastro-intestinal tract are chemically, biologically and, in many instances, functionally distinct from the parent dietary forms. Such features underlie their bio activities and make predictions of effects in mammals including humans extremely difficult to anticipate. Given the enormous variety of flavonoids, the skilled worker has an extremely difficult task in selecting any particular group of flavonoids for successful use in any particular indication. Similarly it has not been possible to make predictions regarding whether a desired effect can be obtained without causing toxicity.

Certain compositions comprising flavonoids possibly suggesting anti-bacterial and even anti-viral activity are known, see for example PCT/GB2007/002756 and PCT/GB2007/002758. De Monbrison et al. have reported the in vitro antimalarial activity of the flavonoid derivatives dehydrosilybin and 8-(1;1)-DMA-kaempferide in Acta Tropica, 97, 2006, 102-107. WO 01/03681 suggests that a vast range of flavonoids can be used against a vast range of infections. However, it provides no data to demonstrate effectiveness against malaria.

Luteolin and quercetin, two major flavonoids, were shown to inhibit the growth of *Leishmania donovani* promastigotes and amastigotes *in vitro* (Mittra, B. et al., Molecular Medicine, 6, 2000, 527-541). These compounds arrest cell cycle progression in L. *donovani* promastigotes, leading to apoptosis.

Quercetin was shown to induce apoptosis of *Trypanosoma brucei gambiense* in cell cultures (Antimicrobial Agents and Chemother., 48, 2004, 924-929). A dose-dependent destruction of the parasites was observed when quercetin was added to *T. b. gambiense* cultures.

The prior art does not demonstrate the use in humans or cattle, sheep, goats, horses, pigs, poultry, dogs or cats of flavonoids for the treatment of internal parasitic infections.

It has now been discovered that a class of flavonoids is effective in treating parasitic infection in a manner which promises significant benefit in the treatment of mammal, especially humans, cattle, sheep, goats, horses, pigs, poultry, dogs and cats. In particular these flavonoids have the desirable quality of being orally administrable.

The parasites for treatment by this invention are internal parasites (endoparasites) or external parasites (ectoparasites).

Internal parasites of interest include those which may be found in the blood, gut, liver, brain, urinary tract or other tissues during part or all of their life cycle. The internal parasites to be treated will generally be those which absorb food from their surroundings in the host (as opposed to having mouth parts that bite food for ingestion). Such internal parasites are generally thin walled so that nutrients are absorbed by diffusion.

Such internal parasites can be multicellular or monocellular parasites and can include worms such as Cestodes such as tapeworm, Trematodes (flukes), flatworms, bladder, blood, liver, lung, kidney and intestinal flukes are another example of multicellular parasites, for example liver fluke. Other flukes such as Fasciola hepatica and Fasciola gigantica, F. halli and F. californica (the latter two in cattle and sheep) are also examples of parasites of interest. Opisthordins sinesis is another fluke that infects humans and O. tencicollis (also called O. felineus), O. viverrini and Paragonimus are also significant.

Of particular use in respect of this invention are single celled parasites particularly protozoa. Protozoa that cause significant disease in humans of relevance to this invention include the causative organisms of malaria, leishmaniasis and trypanosomiasis and various diarrhoeas. Protozoal parasites of relevance include plasmodium species such as P. falciparum, P. vivax, P. ovale, P. malarial, Toxoplasma gondii, Trypanosoma (rhodesiense, gambiense, brucei), T. cruzi and Leishmania spp. Such protozoal infections are often transmitted via insect vectors such as the mosquito, sandfly, tsetse fly and the like. Other protozoal infections of relevance to this invention include coccidiosis (caused by coccidia spp), giardiasis (caused by Giardia spp), babesiosis (caused by Babesia spp) (such diseases more often occurring in domestic animals such as dogs than in humans).

Diarrhoea causing parasites include Crystosporidium parvum, Microsporidia ssp, giardia spp and Entamoeba histolytica. Other pathogens of interest to this invention include Trichomonas vaginalis, Spirochetes, Saporospira, Cristispira and Treponema can variously cause relapsing fever, infective jaundice, Lyme disease, Weil's disease and so are of interest to this invention.

From the foregoing it will be appreciated that protozoa, coccidia, microsporidia, sporozoa, flefellates, cestodes and trematodes cause disease which may be ameloriated by the methods described herein.

External parasites of interest include Lice (anaplura) such as head lice, body lice and pubic lice, particularly (pediculosis humanis capitis), and fleas (cat fleas, dog fleas and human fleas) and scabies.

Up until the present invention, it has not been described to use a bitter orange-derived bioflavonoid in formulations for oral or other administration for therapeutic use against internal parasites or against external parasites.

Several drugs are available for the treatment of parasitic infections such as malaria, interfering with biochemical differences between the parasitic and the human host to attack the former while hoping to minimise toxicity to the latter. Unfortunately, high doses are often required to control or eradicate infection and these produce significant side effects in some people.

Parasitic infections such as malaria are displaying increasing resistance to drugs. This can be the result of, for example, plasmodia expressing molecules such as the Multi-Drug Resistance (MDR) gene which, in most cases, clear the drug molecules from the parasitic cells so that they never reach an effective concentration.

There is therefore a need for new, and preferably less toxic agents to be developed for the treatment of internal or external parasites in mammals such as humans, farmed animals such as poultry, and companion animals.

There is therefore a particular need for new treatments for humans.

The present invention provides a flavonoid-containing composition for systemic, preferably oral, administration for use in the treatment of internal parasites of the types outlined above. Similarly the invention provides certain flavonoid(s) for the treatment of such parasites by systemic, preferably oral, administration. Also this invention provides a method of treatment of parasites by administering to a mammal, aptly to a human or a farmed animal or a companion animal, preferably to a human, in need thereof of certain flavonoid(s).

The present invention similarly provides a flavonoid containing composition for administration for use in the treatment of external parasites either locally or systemically. Such treatments will employ certain flavonoid(s) and may be by, for example, by oral or topical applications, preferably to a human.

The flavonoid containing compositions will comprise at least one compound of the formula (I): 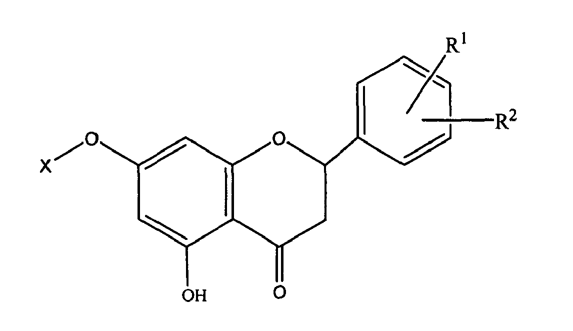 where in R¹ is hydroxyl or methoxyl and R² is hydrogen, hydroxyl or methoxyl and X is hydrogen or a disaccharide.

Aptly R² is hydrogen and R¹ is in the 3- or 4- position.

Aptly R² is hydroxy and R¹ is methoxy, for example, one of R² and R¹ is in the 4- position and the other is in the 3- position, for example R² is in the 4- position and R¹ is in the 3-position.

Favourably R² is hydrogen and R² is 4-hydroxy group.

Favourably R² is a 3-hydroxy group and R¹ is a 4-methoxy group.

Preferably X is a disaccharide.

Suitable disaccharides include combinations of two monosaccharide, suitably pyranoses, linked by a glycosidic bond, for example rhamnose and glucose, for example L-rhamnose and D-glucose.

Suitable disaccharides can have the structure: 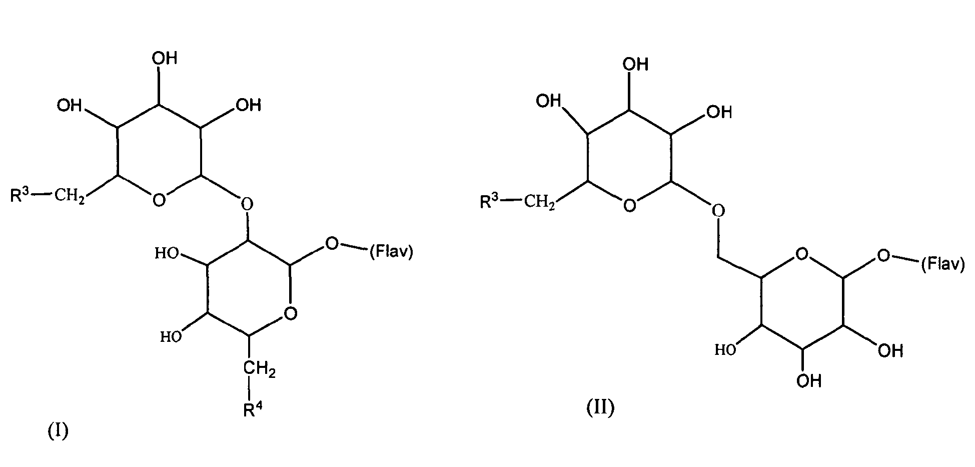 wherein one of R³ and R⁴ is H and the other OH or both are H or both are OH. Aptly R³ is H and R⁴ is OH so that the disaccharide is rutinose.

Favoured aglycones of flavonoids for use in this invention are the disaccharides 6-O-(alpha-L-rhamnopyranosyl)-beta-D-glucopyranose, also known as rutinose, and 2-O-(alpha-L-rhamnopyranosyl)-beta-D-glucopyrarose.

A particularly suitable compound of the formula I is neohesperidin which is of the formula (I) wherein R¹ is 4-methoxy, R² is 4-hydroxy and X is a 2-O-(alpha-L-rhamopyranosyl-beta-D-glucopuranoside).

A further particularly suitable compound of the formula I is naringin which is of the formula (I) wherein R¹ is 4-hydroxy, R² is H and X is rhamnoglucoside.

Suitable compounds of formula (I) include Neoeriocitrin, Isonaringin, Naringin, Hesperidin, Neohesperidin, Neodiosmin, Naringenin, Poncirin and Rhiofolin.

Favoured compositions for use include those which comprise either of naringin and neohesperidin or both.

Particularly aptly the invention will contain naringin and neohesperidin and other flavonoids of the formula (I).

The mixture of flavonoids may aptly contain more than one of neohesperidin, naringin, isocriocrin, isonaringin, hesperidin, neohesperidin, neocliomin, naringenin, poncirin and rhiofolin. Such a mixture of flavonoids can be obtained from bitter oranges, see for example PCT/GB2007/002756. Suitable mixtures can include 2, 3, 4, 5, 6, 7, 8, 9 or more compounds of formula (I). Thus a mixture comprising 2, 3, 5, 6, 7, 8 or 9 of the above named flavonoids is apt, for example containing 3, or containing 4, or containing 5, or containing 6, or containing 7, or containing 8 or containing 9 of said flavonoids.

It is presently believed that mixtures of such flavonoids have advantages over the use of a single flavonoid. It is particularly advantageous that extract of bitter oranges may be employed without the need for isolating individual flavonoids if desired.

Aptly the mixture of flavonoids will comprise at least 25%, more suitably at least 40% and preferably at least 50% of naringin. More aptly the mixture will contain up to 65% of naringin.

Aptly the mixture of flavonoids will comprise at least 15%, more suitably at least 20% and preferably at least 25% of neohesperidin. More aptly the mixture will contain up to 35% of neohesperidin.

In a favoured form the mixture will contain at least 75% of neohesperidin and naringin.

The terms "flavonoids" and "bioflavonoids" are used herein interchangeably.

The compositions will favourably contain an organic acid.

The organic acids may be a di- or tri-carboxylic acid, optionally an aliphatic acid of four to eight carbon atoms and favourably will contain one to four hydroxyl groups. The term includes internal esters of such acids such as ascorbic acid. Favourably the organic acid will be citric acid, malic acid or ascorbic acid or mixtures thereof.

The amount of active agent that may be combined with the carrier material to produce a unit (single) dosage form will vary depending upon the host treated and the particular mode of administration. For example, an oral unit dose, for example a tablet or capsule, or sachet of dry components, optionally for reconstitution e.g. in water, or unit dose solution for humans may contain from 25mg to 5g of flavonoid, for example from 100mg to 2g. Such high doses are possible because of the low toxicity of the compounds. Specific unit doses may contain 125mg, 150mg, 175mg, 200mg, 250mg, 300mg, 400mg, 500mg, 750mg, 1000mg or the like.

Doses for external use will depend upon the area of the body to be treated. Hence for local topical administration to, for example, the head or pubic area, smaller doses will be required than if a large body area needs to be treated, for example, in a widespread infestation by scabies. Generally doses for external application to humans may contain the same doses as set out above for oral dosing.

Doses for farmed or companion animals may be above or below the dose used in humans principally depending on relative body weight. If external parasites such as fleas are to be treated, shampooing the whole animal may be required.

It will be understood, however, that the specific dose level for any particular patient will depend on a variety of factors including the disease to be treated, the age, body weight, general health, sex and diet of the individual being treated; the time and route of administration; the rate of excretion; other drugs which have previously been administered; and the severity of the disease undergoing therapy.

In certain instances, it may be appropriate to administer one of the compositions described herein in combination with another therapeutic agent. By way of example only, the benefit experienced by a patient may be increased by administering one of the compositions described herein with another therapeutic agent (which also includes a therapeutic regimen) that also has therapeutic benefit. By way of example only, in a treatment for malaria involving administration of one of the compositions described herein, increased therapeutic benefit may result by also providing the patient with other therapeutic agents or therapies for malaria. In any case, regardless of the disease, disorder or condition being treated, the overall benefit experienced by the patient may simply be additive of the two therapeutic agents or the patient may experience a synergistic benefit.

The compositions of the present invention may contain hyaluronic acid as described in PCT/GB2007/002756.

The composition of the invention is favourable in the form of an orally administrable unit dose composition for the treatment of a protozoal infection such as malaria.

Pharmaceutical compositions containing the active ingredient may be in any form suitable for the intended method of administration. When used for oral use for example, tablets, troches, lozenges, aqueous or oil suspensions, dispersible powders or granules, emulsions, hard or soft capsules, syrups or elixirs may be prepared. Compositions intended for oral use may be prepared according to any method known to the art for the manufacture of pharmaceutical compositions and such compositions may contain one or more agents including those from the group consisting of sweetening agents, flavouring agents, colouring agents and preserving agents, in order to provide a palatable preparation. Tablets containing the active ingredient in admixture with non-toxic pharmaceutically acceptable excipient which are suitable for manufacture of tablets are acceptable. These excipients may be, for example, inert diluents, such as calcium carbonate, sodium carbonate, lactose, calcium phosphate or sodium phosphate; granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques. Formulations for oral use may be also presented as hard gelatin capsules wherein the active ingredient is mixed with an inert solid diluent, for example calcium phosphate or kaolin, or as soft gelatin capsules wherein the active ingredient is mixed with water or an oil medium, such as peanut oil, liquid paraffm or olive oil.

Powder for reconstitution by addition of water has proved useful. This may be particularly useful if farmed animals are to be treated so that the powder may simply be added to their water supply.

For oral administration, the pharmaceutical formulations may take the form of, for example, tablets or capsules prepared by conventional means with pharmaceutically acceptable excipients such as binding agents (e.g., pregelatinised maize starch, polyvinylpyrrolidone or hydroxypropyl methylcellulose); fillers (e.g., lactose, microcrystalline cellulose or calcium hydrogen phosphate); lubricants (e.g., magnesium stearate, talc or silica); disintegrants (e.g., potato starch or sodium starch glycolate); or wetting agents (e.g., sodium lauryl sulfate). The tablets may be coated by methods well known in the art.

One presently preferred form of orally administrable composition is a solution.

The solutions of the present invention aptly will have a pH in the range of from 3 to 8.5, favorably from 3.5 to 8, more favorably from 4 to 7, and preferably from 5 to 6.5.

Such solutions will contain naringin and/or neohesperidin and optionally other flavonoid derivatives as herein before described.

Such solutions will aptly be formed from a 0.45% to 9% solution of a mixture of bioflavonoids optionally with organic acids or salts thereof; water and a pharmaceutically acceptable carrier. The bioflavonoids may suitably be together with other components of the biomass extracted from the pith of bitter oranges.

Suitably if sodium hyaluronate is present, it has an average molecular weight of between 800,000 and 4,000,000.

The compositions may also aptly comprise choline or a salt thereof, for example with an organic acid such as ascorbic acid.

Especially preferred is when the solution is preparable at the desired pH from water-soluble bioflavonoids in combination with an organic acid, such as citric, malic and ascorbic acids. One or more of the acids are preferably neutralized with a suitable base, such as a quaternary ammonium base, for example a choline base, such as choline carbonate, bicarbonate or, preferably, hydroxide. More preferably, citric, malic and ascorbic acids are all used in the preparation of the composition, and especially preferred is when these are fully neutralized to provide citrate, malate and/or ascorbate salts. Preferably choline is employed as a base or salt. Especially preferred is choline ascorbate. Accordingly, it is preferred that the stock solution is substantially free from unionized organic acids, which is favorably obtained when its pH is around neutral. Exemplary pH ranges for the stock solution are from 3 to 8.5, 3.5 to 8.5, 3.5 to 8, 4 to 8, 4 to 7.5, 4.5 to 7.5, 4.5 to 7, 5 to 7, 5 to 6.5, 5.5 to 6.5 and 5.5 to 6, the pH being for example about 5, about 5.5, about 6, about 6.5 or about 7.

Without wishing to be bound by any particular theory, the present inventors believe that, as well as having a chelating effect on hard water, the organic acids may also synergise the biological activity of the active agent. A preferred solution comprises water-soluble bioflavonoids and choline and ascorbate for example as choline free base together with ascorbic acid or as choline ascorbate.

The solution preferably further comprises a non-toxic solvent, such as a water-miscible or hydrophilic solvent, and more preferably comprises water and a water-miscible co-solvent such as glycerine, propylene glycol or other polyhydric alcohol or the like. Especially preferred is when the solvent comprises a water/glycerine mixture, preferably in the ratio of from 2:1 - 1:2 (water:co-solvent). It is an advantage that such solutions can be alcohol-free, especially ethanol-free.

Accordingly, the stock solution preferably is preparable from:

| **Ingredient** | **% (w/w) Ingredient in Stock Solution** |
|---|---|
| Bioflavonoid mixture (45% in biomass) | 1-20, preferably 2 to 15, more preferably 3 to 15, such as 3, 4, or 15, most preferred is 3.3. |
| Citric acid | 1-20, preferably 4 to 15, such as 4, 5, 10, or 15, most preferred is 4.5. |
| Malic acid | 1-20, preferably 4 to 15, such as 4, 5, 10, or 15, most preferred is 4.5. |
| Ascorbic acid (vitamin C)* | 1-20, preferably 1 to 5*, such as 1, 2, 3, 4, or 5, most preferred is 1.5. |
| Choline hydroxide solution (45% in water)* | 1-45, preferably 4 to 20*, such as 5, 8, 10, 12, 15, or 18. |
| Glycerine/water or other solvent(s) | Balance, qv to 100%, preferably 5-50*, such as 7, 10, or 15, most preferred is 7.5. |

| | |
|---|---|
| * Ascorbic acid and choline hydroxide (or other choline base) can be replaced by choline ascorbate if desired, and if required the amounts of glycerine and water (or alternative solvent(s)) increased appropriately. A preferred solvent comprises about 5% to 25% of glycerine and water, for example approximately equal % of both glycerine and water, such as 15% to 20% glycerine and 15% to 20% water (when choline is present as the hydroxide solution), or such as 25% glycerine and 25% water (when the choline and ascorbic acid are present as 5% choline ascorbate). | |

Accordingly, the compositions of the present invention preferably are preparable from (based on the weight of the composition):
(a) (i) in the range of from 0.0002 - 1.5% w/w bioflavonoids [excluding biomass, which preferably contributes another 0.00024 - 1.83% w/w];
   (ii) in the range of from 0.001 - 2.0% w/w citric acid;
   (iii) in the range of from 0.001 - 2.0% w/w malic acid;
   (iv) in the range of from 0.001 - 2.0% w/w ascorbic acid;
   (v) in the range of from 0.00045 - 2.03% w/w choline base; and
(b), (c) and (d) the balance comprising water, co-solvent(s) and excipient(s) and/or carrier(s).

More preferably, the compositions of the present invention are preparable from (based on the weight of the composition):
(a) (i) in the range of from 0.00045 - 0.9% w/w bioflavonoids [excluding biomass, which preferably contributes another 0.00055 - 1.1% w/w];
   (ii) in the range of from 0.001 - 2.0% w/w citric acid;
   (iii) in the range of from 0.001 - 2.0% w/w malic acid;
   (iv) in the range of from 0.001 - 2.0% w/w ascorbic acid;
   (v) in the range of from 0.00045 - 2.03% w/w choline base; and
(b), (c) and (d) the balance comprising water, co-solvent(s) and excipient(s) and/or carrier(s).

Since the stock solutions of the present invention therefore more preferably are preparable from the percentages given in the above-noted table, the compositions of the present invention more preferably are preparable from:
(a) (i) in the range of from 0.000675 - 0.675% w/w bioflavonoids [excluding biomass];
   (ii) in the range of from 0.015 - 1.5% w/w citric acid;
   (iii) in the range of from 0.015 - 1.5% w/w malic acid;
   (iv) in the range of from 0.005 - 0.5% w/w ascorbic acid;
   (v) in the range of from 0.015 - 0.9% w/w choline base; and
(b), (c) and (d) the balance comprising water, co-solvent(s) and excipient(s) and/or carrier(s).

Since preferred compositions of the present invention comprise in the order of 1% w/w of the stock solution, in one embodiment, preferred compositions of the invention are preparable from:
(a) (i) of the order of 0.0675% w/w bioflavonoids [excluding biomass];
   (ii) of the order of 0.15% w/w citric acid;
   (iii) of the order of 0.15% w/w malic acid;
   (iv) of the order of 0.05% w/w ascorbic acid;
   (v) of the order of 0.09% w/w choline base; and
(b), (c) and (d) the balance comprising water, co-solvent(s) and excipient(s) and/or carrier(s).

In another embodiment, most preferred compositions of the invention are preparable from:
(a) (i) of the order of 0.01485% w/w bioflavonoids [excluding biomass];
   (ii) of the order of 0.045% w/w citric acid;
   (iii) of the order of 0.045% w/w malic acid;
   (iv) of the order of 0.015% w/w ascorbic acid; and
(b), (c) and (d) the balance comprising water, co-solvent(s) and excipient(s) and/or carrier(s).

In another embodiment, most preferred compositions of the invention are preparable from:
(a) (i) of the order of 0.01485% w/w bioflavonoids [excluding biomass];
   (ii) of the order of 0.045% w/w citric acid;
   (iii) of the order of 0.045% w/w malic acid;
   (iv) of the order of 0.06% w/w choline ascorbate; and
(b), (c) and (d) the balance comprising water, co-solvent(s) and excipient(s) and/or carrier(s).

The stock solution may be prepared by processes known to those skilled in the art. Preferably, the co-solvents are mixed with the water at ambient temperature and then the acids involved in neutralization processes, such as ascorbic acid, are blended together with the solvent at an increased temperature, which is kept low enough to ensure no degradation of any of the ingredients. In the case of ascorbic acid, which thermally degrades above about 55 degC, the temperature is kept in the range of from about 25 to below 55 degC and is preferably in the region of 50 degC. Preferably, the neutralization involves addition of choline hydroxide to ascorbic acid in the blend (starting pH = 1.2; finishing pH = 5.5-6.0), or choline ascorbate (ie wherein the ascorbic is already neutralized) itself can be added.

Then, the remaining acids (preferably, citric and malic) are added, followed by the bioflavonoids, resulting in a solution having a pH in the range of from about 2.0 to 6.5 but typically is from about 2.2 to 3.5, especially in the range of from 2.3 to 3.0. The remaining un-neutralised acids are also substantially neutralized, for example, by choline hydroxide, to result in a substantially neutral solution having a pH in the range of, for example, from 5 to 8.5, preferably 5.5 to 7, more preferably 5.5 to 6.5.

Aptly, the bioflavonoid mixture comprises water-soluble bioflavonoids in association with biomass resulting from the extraction process; accordingly, the bioflavonoid mixture may be associated with up to 40-60% w/w, preferably about 55% w/w, biomass (based on the weight of the bioflavonoid mixture). The flavonoids are preferably glucosides, especially those selected from Neoeriocitrin, isonaringin, naringin, hesperidin, neohesperidin, Neodiosmin, Naringenin, poncirin and Rhiofolin, and more preferably each of these is present in the mixture. Especially preferred is when the major part of the bioflavonoid mixture (i.e. more than 50%) comprises naringin and neohesperidin, such as when these comprise in excess of 75% of the bioflavonoid component (excluding biomass). Suitably, other bioflavonoids (such as flavonol, chrysin, hesperetin) are substantially absent from the bioflavonoid mixture and the bioflavonoid component therefore consists essentially of the water-soluble bioflavonoids listed hereinabove, although trace amounts of other bioflavonoids may be present. Especially preferred is when the water-soluble bioflavonoids comprise the following percentages (by weight of bioflavonoid in the total bioflavonoid component):

| **Bioflavonoid** | **% of Total Bioflavonoid Component** |
|---|---|
| Neoeriocitrin | 2.4 |
| Isonaringin | 2.7 |
| Naringin | 52.0 |
| Hesperidin | 3.1 |
| Neohesperidin | 27.8 |
| Neodiosmin | 3.1 |
| Naringenin | 3.4 |
| Poncirin | 4.4 |
| Rhiofolin | 1.1 |
| **Total** | **100%** |

A suitable source of such a water-soluble bioflavonoid mixture is herein referred to as 'HPLC 45', of which about 45% (of the total composition of HPLC 45) comprises such bioflavonoids, with the balance (about 55%) comprising biomass such as pectins, sugars and minor organic acids. As stated above, especially preferred is when the major part of the bioflavonoid mixture comprises naringin and neohesperidin, such as when these comprise in excess of 35% of the bioflavonoid component in a mixture with biomass such as HPLC 45. Accordingly, by weight of the total composition of HPLC 45, the following bioflavonoids are preferably present:

| **Bioflavonoid** | **% in HPLC 45 (bioflavonoid component + biomass)** |
|---|---|
| Neoeriocitrin | 1.1 |
| Isonaringin | 1.2 |
| Naringin | 23.4 |
| Hesperidin | 1.4 |
| Neohesperidin | 12.5 |
| Neodiosmin | 1.4 |
| Naringenin | 1.5 |
| Poncirin | 2.0 |
| Rhiofolin | 0.5 |
| **Total** | **45% of HPLC 45** |

The HPLC 45 is available from Exquim (the food arm of Grupo Ferrer) as Citrus Bioflavonoid Complex 45% HPLC. It is derived from a starting material comprised of the pith of immature, bitter (blood/red) oranges such as Seville oranges that are classed as 'inedible' and from which the pips, flesh and oily skin have been substantially removed or remain undeveloped. This starting material is crushed in a hydrophilic, ionic solvent such as water or water/alcohol mixtures, preferably water/ethanol in a ratio of about 1:10-20 (solvent: starting material). The resulting mixture is filtered to leave a water-soluble biomass, which is retained, and an insoluble biomass, which is discarded. The water-soluble biomass is then subject to fine filtration, after which it is flash-distilled to leave a brown, hygroscopic powder (HPLC 45).

Preferably, the bioflavonoid mixture for use in the compositions of the present invention comprises water-soluble glucosides from the mixture obtained from bitter oranges (Bergamot) or other citrus fruits or other plant sources, which comprise water-insoluble flavonoids of formula (I); and, more preferably, is from the mixture obtained when substantial amounts of the seeds, pulp and/or flesh of such fruits are comprised in the starting material, which particularly comprise water-insoluble components.

Preferably, the stock solution comprises 1-20%, preferably 2 to 15%, more preferably 3 to 15%, such as 3, 4, or 15, most preferred is 3.3% w/w of the HPLC45. Accordingly, the stock solution preferably comprises 0.45-9%, preferably 0.9 to 6.75%, more preferably 1.35 to 6.75%, such as 1.35, 1.8, or 6.75, most preferred is 1.485% of the bioflavonoid mixture.

Preferably, the compositions for use in the invention and, particularly in the absence of other ingredients except water, the stock solution, has a pH of from about 3 to about 8.5, more preferably of from about 4 to 7.5, such as about 5 to 7 ; especially preferred is when the pH is about 5.5 to 6.5. Most preferably, therefore, the organic acids used in the preparation of the stock solution and/or composition therefore have been substantially neutralised, preferably as described above by addition of a base to the stock solution. On the other hand, when the composition also comprises a buffering agent, then the pH of the stock solution can vary outside these ranges provided that the buffering agent is present in an amount effective to provide the composition with a pH within these ranges.

Accordingly, the solutions for use in this invention may comprise a buffering agent to regulate or adjust the pH of the final composition, such as an alkali metal hydroxide or ammonium hydroxide or a mono-, di- or tri-basic phosphate such as a tri(alkali metal) phosphate. Since the quantity of hydroxide is more difficult to measure than that of dibasic phosphate, it is preferred to use monobasic phosphates and dibasic phosphates. Another alternative is to use a combination of phosphoric acid with a dibasic or tribasic, such as tri(alkali metal), phosphate. The phosphates are preferably incorporated in the form of their sodium, potassium or ammonium salts; more preferably, sodium salts are employed. However, in cases where hypertensive effects of sodium ions are of concern, mono- and di-potassium phosphates may be used. When the buffering agent is disodium phosphate, for example, it may be present up to about 5% w/w of the composition, preferably in the range of from 0 to 0.5%, such as about 0.05% w/w.

Other additives may be present in the solutions for use in the invention, such as flavouring, sweetening or colouring agents, or preservatives. Mint, such as from peppermint or spearmint, cinnamon, eucalyptus, citrus, cassia, anise and menthol are examples of suitable flavouring agents. Flavouring agents are preferably present in the oral compositions in an amount in the range of from 0 to 3%; preferably up to 2%, such as up to 0.5%, preferably around 0.2%, in the case of liquid compositions. Sweeteners include artificial or natural sweetening agents, such as sodium saccharin which may be present in an amount in the range of from 0 to 2%, preferably up to 1% w/w, such as 0.05 to 0.3% w/w of the oral composition. Colouring agents are suitable natural or synthetic colours, such as titanium dioxide or CI 42090, or mixtures thereof. Colouring agents are preferably present in the compositions in an amount in the range of from 0 to 3%; preferably up to 0.1%, such as up to 0.05%, preferably around 0.005-0.0005%, in the case of liquid compositions. Of the usual preservatives, sodium benzoate is preferred in concentrations insufficient substantially to alter the pH of the composition, otherwise the amount of buffering agent may need to be adjusted to arrive at the desired pH.

Other optional ingredients of the solutions for use in this invention may include humectants, surfactants (non-ionic, cationic or amphoteric), thickeners, gums and binding agents. Suitable humectants include glycerine, xylitol, glycerol and glycols such as propylene glycol, which may be present in an amount of up to 50% w/w each, but total humectant is preferably not more than about 60-80% w/w of the composition. For example, liquid compositions may comprise up to about 30% glycerine plus up to about 5%, preferably about 2% w/w xylitol. Surfactants are preferably not anionic and may include polysorbate 20 or cocoamidobetaine or the like in an amount up to about 6%, preferably about 1.5 to 3%, w/w of the composition.

Preferably, the compositions in the form of solutions are packaged in suitable packaging such as a plastics or metallic tube, plastics or glass transparent, translucent or opaque bottle, jar or dispenser, together with instructions for use. Such packaging may itself be further packaged into a cardboard box or other suitable container and the same or further instructions for use may be inserted therein or inscribed thereon; suitably, such instructions may be inscribed on a pack insert or leaflet. The packaging preferably lists the active, main or all ingredients of the composition. The instructions may include those known to the person skilled in the art of compositions, particularly those for anti-parasitic use. Solid compositions, for example tablets, can be packed in blister packs, reclosable containers or the like.

In the treatment of parasitic diseases, for example protozoal disease such as malaria in humans (or other host), the flavonoids of the invention may be administered, for example, from 1 to 6 times a day, generally 1, 2, 3 or 4 times a day. Administration once or twice a day favours patient compliance.

Such use may be prophylactic (to prevent development of the disease) or therapeutic (to ameliorate or cure the disease after it has been acquired).

The flavonoids may be administered from the first symptom of disease until, for example, one week after the last symptom abates to treat the disease. Alternatively for prophylactic use the flavonoids may be administered from first entry of a patient to a site of potential infection to one week after leaving the site of potential infection.

Alternatively, blood samples may be analysed and any patient whose blood shows evidence of disease may be treated by administration of flavonoids of the invention.

It is an advantage of the present invention that at least some chloroquine-resistant strains of malaria may be treated. Use in the treatment of MDR strains is also contemplated.

It is an advantage of the invention that withdrawal of treatment does not result in an immediate resurgence of parasitaemia (which indicates the treatment has an irreversible effect directly on the parasite).

It is an advantage of the invention when treating malaria that development to maturity of the malaria parasite in red blood cells is disrupted and that invasion of the red blood cells by malaria parasites is reduced or substantially eliminated.

Use of flavonoids for 24 hours, more aptly 48 hours, and favourably for 76 hours, prevents or at least substantially reduces schizont formation and/or prevents new ring stages forming a new cycle of infection. The flavonoids hence halve the life cycle of the parasite and the cycle of re-infection.

The flavonoids may therefore be employed to kill malarial parasites (rather than to suppress them) so that infection (in the absence of a new vector-induced infection) is unlikely to occur, for example, in the following 30 days or even possibly permanently.

The flavonoids may be used prophylactically to prevent development of the disease in the event of infection by the vector. This may be performed by administering the composition on an ongoing basis (for example 1, 2, 3 or 4 times a day) during the period of potential exposure to the vector. Alternatively less frequent dosing, for example, every 2 or 3 days or even once a week, may be envisaged.

Other parasitic diseases, particularly protozoal caused diseases such as those caused by Leishmanial or Trypanosomal parasites may be treated in an analogous manner, as can diseases caused by parasitic flukes, worms and the like.

The invention provides a method of treatment or prophylaxis of disease caused by an internal parasite which involves administering to a mammal an effective amount of a flavonoid of formula (I).

Suitably the mammal being treated is a human. Suitably the administration to the mammal is orally. The diseases to be treated, the routes of administration, the pharmaceutical compositions and the flavonoids and mixtures thereof employed are as described above. The disease is most aptly malaria.

For the treatment of external parasites suitable compositions may be formulated as solutions (lotions), gels, shampoos, soaps and the like.

Suitable solutions may contain typical carriers suitable for use in medical products for the elimination of head lice or other parasites. Favourably agents such as humectant such as polyhydric liquid alcohols such as glycerine, propylene glycol or butylene glycol may be employed. Monohydric alcohols such as ethanol and propanol may also be employed of which iso-propanol is particularly apt. Preferably a solution in a mixture of water, isopropanol and glycerol may be employed. Such compositions may contain perfumes and the like, for example an extract of aloe vera. Certain compositions may be presented in the form of soaps or shampoos by the inclusion of surfactants and/or foam enhancers.

Generally compositions for the treatment of external parasites may contain a mix of bioflavanoids, for example 50 - 55% of naringin, 25 - 30% of neohesperidin and 15 - 25% of other bioflavanoids of Formula (I).

Other additives may be employed as described generally herein for use in compositions.

The present invention will now be illustrated by the following examples.

### Example 1: Preparation of Stock Solution

### (a) Preparation of HPLC 45

The starting material comprises the pith of immature, bitter (blood/red) oranges such as Seville oranges that are classed as 'inedible' and from which the pips, flesh and oily skin have been substantially removed. The starting material is milled and then crushed in water or water/ethanol in a ratio of about 1:10-20 (solvent: starting material). The resulting mixture is filtered to leave a water-soluble biomass, which is retained, and an insoluble biomass, which is discarded. The water-soluble biomass is then subject to fine filtration, after which it is flash-distilled to leave a brown, hygroscopic powder (HPLC 45). Alternatively, the HPLC 45 is available from Exquim (Grupo Ferrer).

### (b) Bioflavonoid Composition of HPLC 45

Analysis of the HPLC 45 obtained in step (a) shows that 45% of the total composition of HPLC 45 comprises bioflavonoids, with the balance (55%) comprising pectins, sugars and minor organic acids. The percentage (by weight of bioflavonoids in the HPLC 45) of the following bioflavonoids are present:

| **Bioflavonoid** | **% Bioflavonoids in HPLC 45** |
|---|---|
| Neoeriocitrin | 2.4 |
| Isonaringin | 2.7 |
| Naringin | 52.0 |
| Hesperidin | 3.1 |
| Neohesperidin | 27.8 |
| Neodiosmin | 3.1 |
| Naringenin | 3.4 |
| Poncirin | 4.4 |
| Rhiofolin | 1.1 |
| **Total** | **100%** |

Accordingly, by weight of the total composition of HPLC 45, the following bioflavonoids are present:

| **Bioflavonoid** | **% HPLC 45** |
|---|---|
| Neoeriocitrin | 1.1 |
| Isonaringin | 1.2 |
| Naringin | 23.4 |
| Hesperidin | 1.4 |
| Neohesperidin | 12.5 |
| Neodiosmin | 1.4 |
| Naringenin | 1.5 |
| Poncirin | 2.0 |
| Rhiofolin | 2.8 |

### (c) Preparation of Stock Solution

| **Ingredient** | **% Stock Solution** |
|---|---|
| HPLC 45 | 15 |
| Citric acid | 15 |
| Malic acid | 15 |
| Ascorbic acid (vitamin C)* | 5* |
| Choline hydroxide solution (45% in water)* | 15* |
| Glycerine | 15* |
| Water | 20* |
| **Total** | **100%** |

The water, glycerine and ascorbic acid are blended together at ambient temperature and the temperature then increased to 50 degC. The choline hydroxide is added to neutralize the ascorbic acid (starting pH = 1.2; finishing pH = 5.5-6.0).

### [* Ascorbic acid and choline hydroxide can be replaced by choline ascorbate 5%, with amounts of glycerine and water increased to 25% each]

Then, the remaining acids (citric and malic) are added, followed by the HPLC 45, resulting in a stock solution having a pH of 6.2 to 7.2, and comprising 6.75% bioflavonoids (w/w of the stock solution).

### Example 2: Preparation of Solution

The following solution was prepared as above:

| | |
|---|---|
| Bioflavonoid mix | 3.3% |
| Malic acid | 4.5% |
| Citric acid | 4.5% |
| Glycerin | 7.5% |
| Ascorbic acid | 1.5% |
| Water | 78.6% |
| pH of solution | 1.5 to 1.75 |

### Example 3(a): Preparation of Solution

The following solution was prepared as above:

| | |
|---|---|
| Bioflavonoid mix | 3.3% |
| Malic acid | 4.5% |
| Citric acid | 4.5% |
| Choline ascorbate | 6.0% |
| LFG61 alkyl glycoside | 13.3% |
| Propylene glycol | 7.5% |
| Water | 60.9% |
| pH of solution | 1.5 to 1.75 |

### Example 3(b): Preparation of Solution

| Ingredient | % Stock Solution |
|---|---|
| HPLC 45 | 15 |
| Citric acid | 10 |
| Malic acid | 15 |
| Ascorbic acid (vitamin C) | 5 |
| Choline hydroxide solution (45% in water) | 15 |
| Glycerine | 15 |
| Water | 15 |
| Tyrosol | 10 |
| Total | 100% |

### Example 4: Antimalarial Activity

MDC refers to the composition of Example 3(a) and MDCH refers to the composition of Example 3(b).

The activity of compositions of the invention against malaria was determined *in vitro* and *in vivo.*

### In vitro assay

On testing *in vitro* activity of MDC against the human infective form of malaria, *Plasmodium falciparum.*

MDC was able to kill *Plasmodium falciparum* parasitics even when diluted several thousand times. The dilution required to kill 50% of the parasitics (the ED50 value) was almost 6000-fold for MDC and 2500-fold for MDCH.

### In vivo assay

When mice infected with *Plasmodium sp.* drank a solution of MDC, there was a significant reduction in the number of parasitics in the blood. The animals' condition was good, with no signs of dehydration or toxicity

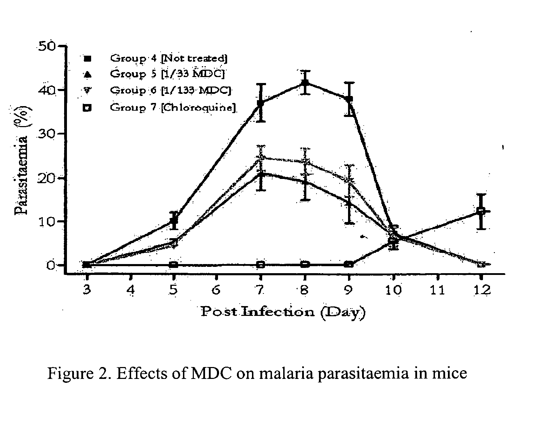

Figure 2 shows the effects of MDC on malaria parasitaemia in mice. When mice were infected with *Plasmodium chabaudi,* MDC diluted 1:33 produced a 50% reduction in parasite counts even in the first wave of parasitaemia, compared with untreated mice.

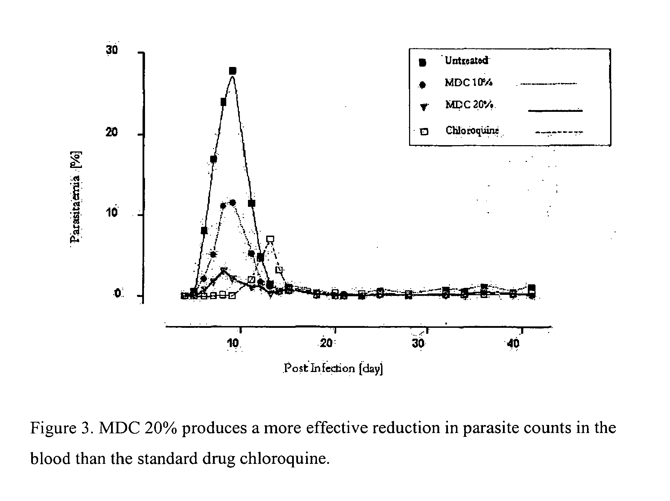

Figure 3 shows the effects of a 1:5 and a 1:10 dilution of MDC on malaria parasites in mice. A 1:5 dilution of MDC (20%) produced a more effective reduction in parasite counts in the blood than the standard drug chloroquine. The 20% concentration was still well tolerated by the test animals, with no signs of discomfort, side effects or loss of weight. The animals continued to eat and drink normally. These results show that at this concentration, MDC almost totally eliminated the parasites from the blood and proved to be more effective than the chloroquine, an anti-malarial drug used routinely in humans.

### Example 5: Antileishmaniasis Activity

The activity of compositions of the invention against leishmania parasites was determined *in vitro.*

MDC refers to the composition of Example 3.

### In vitro assay

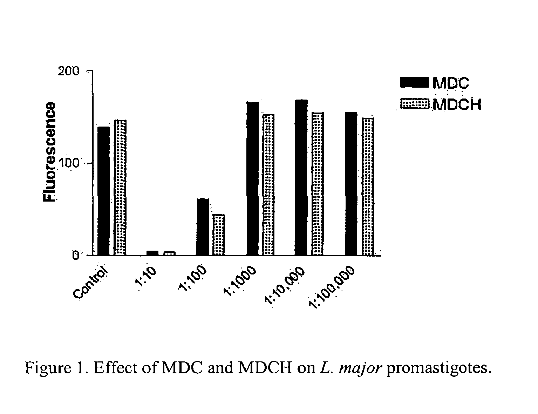

Figure 1 shows the *in vitro* activity of MDC and MDCH against *Leishmania major* promastigotes. MDC and MDCH kill insect-stage (promastigotes) leishmania parasites *in vitro* and both MDC and MDCH display *in vitro* activity against leishmania parasites in dilute solution.

### Example 6: Antitrypanosomiasis Activity

The activity of compositions of the invention against trypanosomal parasites was determined *in vitro* and *in vivo.*

MDC refers to the composition of Example 3 and MDCH refers to a more concentrated version.

### In vitro assay

On testing *in vitro* activity of MDC and MDCH against wild type and drug-resistant strains of trypanosomal parasites.

MDC and MDCH were able to kill trypanosomal parasites *in vitro* even when diluted several thousand times. Both flavonoid-containing compositions were effective against strains of trypanosomes which are resistant to the drugs currently available (MDR strains), indicating that MDC is not cross-resistant with the current drugs.

### In vivo assay

In mice infected with *Trypanosoma brucei,* parasitaemia was much delayed in the mice protected by MDC and several mice stayed completely free from infection.

### Example 7

The following composition was prepared comprising:-

| | **% w/w** |
|---|---|
| Bioflavanoid mix | 0.5 |
| Glycerin | 1.0 |
| Isopropanol | 30.0 |
| Aloe Vera Extract | 0.1 |
| Natrasol | 0.65 |
| Water | 67.75 |

When tested again head lice the formulation achieved a 100% kill rate. This composition may be applied to the hair of a subject infested with head lice or to a subject in danger of becoming infested.

## Claims

**1.** A flavonoid of formula (I) 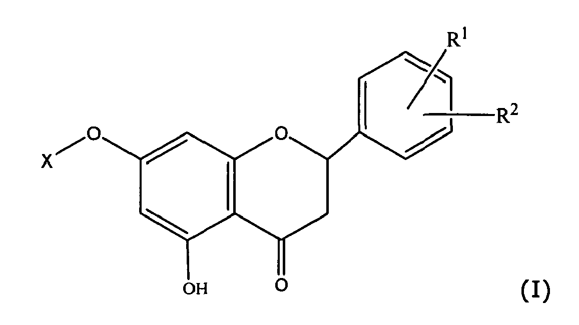 for use in the treatment of parasitic infection, wherein wherein R¹ is hydroxyl or methoxyl and R² is hydrogen, hydroxyl or methoxyl and X is hydrogen or a disaccharide.

**2.** A flavonoid for use as claimed in claim 1 wherein the treatment is by oral administration for treatment of internal parasites or by external administration for treatment of external parasites.

**3.** A flavonoid for use as claimed in claims 1 or 2 wherein R² is hydrogen and R¹ is in the 3- or 4- position or R² is 3-hydroxy and R¹ is 4-methoxy.

**5.** A flavonoid for use as claimed in claim 1 which is naringin or neohesperidin.

**6.** A flavonoid for use as claimed in any of claims 1 to 5 in mixture with other flavonoids of a mixture of flavonoids extracted from the pith of bitter oranges, particularly with one or more of Neoeriocitrin, Isonaringin, Naringin, Hesperidin, Neohesperidin, Neodiosmin, Naringenin, Poncirin and Rhiofolin.

**7.** A pharmaceutical composition for use in the treatment of an internal parasitic infection which comprises a flavonoid or mixture of flavonoids as described in any of claims 1 to 6.

**8.** A flavonoid for use or a composition for use as claimed in any of claims 1 to 7 for the treatment or prophylaxis of a disease caused by internal parasites that obtain food from the host by absorption.

**9.** A composition as claimed in claim 1 in the form of an orally administratable unit dose.

**10.** A flavonoid for use or a composition for use as claimed in any of claims 1 to 8 for the treatment or prophylaxis of a protozoal infection.

**11.** A flavonoid for use or a composition for use as claimed in claim 9 for the treatment of malaria in humans.

**12.** A flavonoid for use or a composition as claimed in claim 9 for the prophylaxis of malaria.

**13.** A method of treating or prophylaxis of a protozoal infection, particularly malaria, which comprises the oral administration to a subject in need thereof of an effective amount of a flavonoid or composition as described in any of claims 1 to 8.

**14.** A method of treating or prophylaxis of an infestation by lice, particularly head lice, which comprises treating a subject in need thereof of an effective amount of a flavonoid or composition as described in any of claims 1 to 8.

**15.** A method as claimed in claim 14 wherein the treatment comprises the external treatment with a shampoo.
